# EUROPEAN PATENT SPECIFICATION

(11) **EP 1 663 247 B1**
(45) Date of publication and mention of the grant of the patent: **28.10.2009**
(21) Application number: 04764755.7
(22) Date of filing: 02.09.2004
(51) Int. Cl.: A61K 31/55

(54) **USE OF OXCARBAZEPINE FOR THE IMPROVEMENT OF SLEEP IN PATIENTS SUFFERING FROM CHRONIC PAIN**
VERWENDUNG VON OXCARBAZEPIN ZUR VERBESSERUNG DES SCHLAFES BEI PATIENTEN DIE AN CHRONISCHEM SCHMERZ LEIDEN
UTILISATION D'OXCARBAZEPINE DANS L'AMELIORATION DU SOMMEIL CHEZ DES PATIENTS SOUFFRANT DE LA DOULEUR CHRONIQUE

(30) Priority: 03.09.2003 GB 0320637; 16.01.2004 US 537378 P
(43) Date of publication of application: 07.06.2006
(73) Proprietor: Novartis AG, 4056 Basel (CH); Novartis Pharma GmbH, 1230 Wien (AT)
(72) Inventor: MANNING, Donald, Bloomsbury, NJ 08804 (US)
(74) Representative: Harrison Goddard Foote
(86) International application number: PCT/EP2004/009797
(87) International publication number: WO 2005/020968

(56) References cited:
- WO-A-01/32183
- WO-A-2004/035041
- CARRAZANA E; MIKOSHIBA I: "Rationale and evidence for the use of oxcarbazepine in neuropathic pain" JOURNAL OF PAIN AND SYMPTOM MANAGEMENT, vol. 25, no. 5, 1 May 2003 (2003-05-01), pages S31-S35, XP009042848
- SCHMADER KENNETH E: "Epidemiology and impact on quality of life of postherapetic neuralgia" THE CLINICAL JOURNAL OF PAIN, vol. 18, no. 6, 2002, pages 350-354, XP009042872

## Description

The present invention relates to novel uses of the carbamazepine derivative of formula I 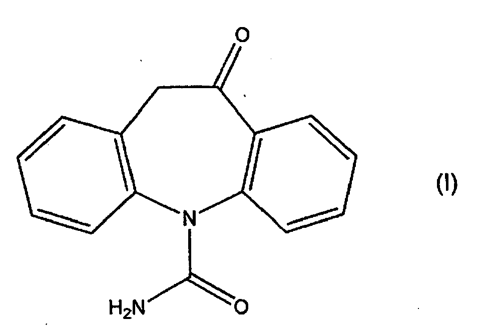 and its pharmaceutically acceptable salts, for the improvement of sleep in human patients suffering from chronic pain.

The compound of formula I is known as "oxcarbazepine" (10-oxo-10,11-dihydro-5H-dibenz[b,f]azepine-5-carboxamide) and is, eg., marketed under the brand name Trileptal^{®}.

Oxcarbazepine is a known anticonvulsant drug useful in the treatment of seizures of, for example, epileptic origin. Its preparation is described, e.g., in the US patent 3,642,775 and WO 01/56992.

It was surprisingly found that sleep can be improved in human patients suffering from chronic pain, in particular neuropathic pain, especially diabetic neuropathic pain, by decreasing the frequency of being awakened from sleep due to pain, decreasing the delay of getting to sleep due to pain or feeling rested after sleep by administering oxcarbazepine, especially according to one of the dosing regimens of the present invention.

According to one embodiment, the present invention relates to a method of improving sleep in human patients suffering from chronic pain, in particular neuropathic pain, especially diabetic neuropathic pain, which comprises administering oxcarbazepine or a pharmaceutically acceptable salt thereof to said patient especially on a twice daily schedule, at a total dose in the range from about 450 mg/day to about 900 mg/day, particularly a total dose of about 550mg/day to about 750 mg/day, especially 600 mg/day.

According to another embodiment, the present invention relates to a method of improving sleep in human patients suffering from chronic pain, in particular neuropathic pain, especially diabetic neuropathic pain, which comprises administering oxcarbazepine or a pharmaceutically acceptable salt thereof to said patient especially on a twice daily schedule, at a total dose in the range from about 900 mg/day to about 1500 mg/day, particularly a total dose of 1050 mg/day to 1350 mg/day, especially 1200 mg/day.

According to another embodiment, the present invention relates to a method of improving sleep in human patients suffering from chronic pain, in particular neuropathic pain, especially diabetic neuropathic pain, which comprises administering oxcarbazepine or a pharmaceutically acceptable salt thereof to said patient especially on a twice daily schedule, at a total dose in the range from about 1500 mg/day to about 2100 mg/day, particularly a total dose of 1650 mg/day to 1950 mg/day, especially 1800 mg/day.

The term "neuropathic pain" as used herein includes, but is not restricted to, pain that frequently accompanies nerve damage resulting from a range of pathologies including amputation or conditions such as diabetes, post-herpetic neuralgia or trigeminal neuralgia. The hyperalgesia and allodynia associated with neuropathic pain is particularly intractable and poorly treated in the clinic by treatments such as opiates or non-steroidal anti-inflammatory drugs.

The usefulness of oxcarbazepine in the treatment of the above-mentioned disorders including the improvement in sleep quality can be confirmed in suitable clinical studies, e.g. those described in the Examples, e.g. applying a total daily dosage of 600 mg, 1200 mg or 1800 mg oxcarbazepine. The person skilled in the pertinent art is fully enabled to select a relevant test model to prove such usefulness. Suitable clinical studies are in particular randomized, double-blind, placebo-controlled, parallel studies in neuropathic pain patients.

### Short Description of the Figures

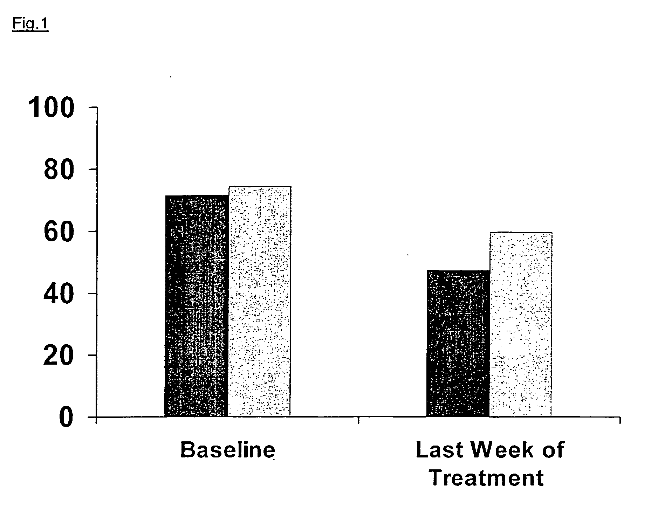

Fig. 1 depicts the average VAS score (y-axis) during the last week of treatment of the clinical study described in Example 1. The darker area represents the scores obtained in the group of patients obtaining oxcarbazepine at a daily dosage of 1800 mg (N=68). The brighter area represents the score for the placebo group (N=76).

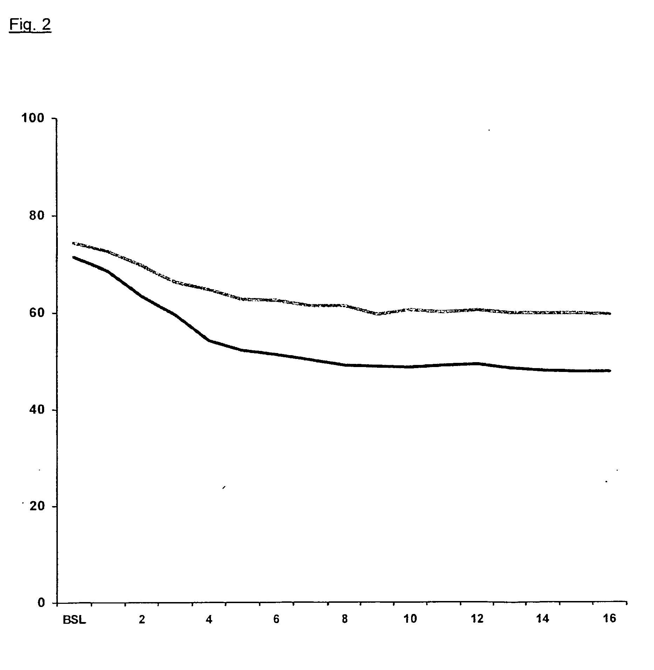

Fig. 2 illustrates the average VAS score (y-axis) by week (x-axis) of the clinical study described in Example 1. The upper line represents the score for the placebo group (N=76). The lower line represents the scores obtained in the group of patients obtaining oxcarbazepine at a daily dosage of 1800 mg (N=68).

According to one aspect of the present disclosure, oxcarbazepine is given twice daily on a continuous basis, alone, or during and subsequent to other therapies, for example during the treatment of diabetes.

The single doses applied can range between 150 and 1200 mg, eg. 300 mg, 600 mg or 900 mg, of oxcarbazepine. For instance, in one embodiment of the invention, two single doses of about 900 mg are applied 6 to 12 hours apart, for example about 8 hours apart.

Oxcarbazepine may be administered in any usual manner, eg. orally, for example in the form of tablets or capsules, or parenterally, for example in the form of injection solutions or suspensions.

In one embodiment of the invention, the pharmaceutical composition is preferably a tablet, more preferably a tablet as disclosed in US 4,353,887 or, most preferably, a film-coated tablet, eg. as described in WO 98/35681.

In another embodiment of the invention, the pharmaceutical composition is preferably an oral suspension, more preferably an oral suspension as disclosed in WO 01/45671.

Unit dosage forms may contain, for example, from about 2.5 mg to about 1000 mg of oxcarbazepine eg. 150 mg or 300 mg.

The invention additionally provides for the use of oxcarbazepine for the manufacture of a pharmaceutical composition for the improvement of sleep in human patients suffering from chronic pain, in particular neuropathic pain, especially diabetic neuropathic pain.

Furthermore, the present disclosure includes
- a package comprising a pharmaceutical composition comprising as sole active ingredient oxcarbazepine together with instructions for improvement of sleep in human patients suffering from chronic pain, in particular neuropathic pain, especially diabetic neuropathic pain; and
- a pharmaceutical composition comprising as sole active ingredient oxcarbazepine for the improvement of sleep in human patients suffering from chronic pain, in particular neuropathic pain, especially diabetic neuropathic pain.

### EXAMPLE 1 - Clinical Study in Human Diabetic Neuropathic Pain Patients

Although this example does not form part of the invention as claimed, it is retained as useful background information.

In a clinical study in a Caucasian patient population, a full double-blind treatment was conducted for a period of 112 days. Patients suffering from a diabetic neuropathic pain obtained placebo or oxcarbazepine. Oxcarbazepine at a total dosage of 1800 mg/day was statistically significantly superior to placebo with respect to the average Visual Analog Scale (VAS) for pain severity score during the last week of double-blind treatment (p=0.0108). Furthermore, the group treated with oxcarbazepine at a total dose of 1800 mg/day had a statistically significantly higher percentage of responders (those felt slightly, much, or very much improved) (73.2%) compared with the placebo group (39.7%) (p=0.0003).

### EXAMPLE 2 - Impact of Oxcarbazepine on Sleep in Human Diabetic Neuropathic Pain Patients

In a multicenter, placebo-controlled, double-blind, parallel-group study the efficacy of oxcarbazepine up to 1800 mg/day in patients with neuropathic pain of diabetic origin was evaluated. The study consisted of three phases: a pre-randomization screening phase (2 weeks); a double-blind treatment phase (18 weeks); and an open-label extension phase (52 weeks). The double-blind treatment phase was further divided into a titration period of 4 weeks, maintenance period of 12 weeks, and a follow-up period of 2 weeks. Patients who met all inclusion criteria were random ized in a 1:1 ratio to receive either study medication or placebo during double-blind treatment (16 weeks). After randomization into the double-blind treatment phase, oxcarbazepine or placebo was initiated at 300 mg/day, and increased 3 days later to 300 mg twice a day (600 mg/day). After this, oxcarbazepine was titrated as tolerated up to a maximum target dose of 900 mg twice a day (1800 mg/day) in increments of 300 mg every 5 days over the 4-week titration period. During the remaining 12 weeks of the study (maintenance period), oxcarbazepine treatment remained at the dose reached by day 28. Patient response to the following three questions from the daily sleep questionnaire were used to assess disturbances of sleep over double-blind treatment: 1) Did your pain delay you in getting to sleep last night? 2) Did your pain awaken you from sleep more than one time last night? 3) Did you feel rested after sleep this morning?

The mean proportions of days that patients were awakened during the night due to pain was lower in oxcarbazepine-treated patients compared with the placebo group (31% vs 49% of study days; P=0.02) (Table 1). In addition, a lower proportion of oxcarbazepine-treated patients experienced delays in getting to sleep and a greater proportion of oxcarbazepine-treated patients felt rested after sleep in comparison with placebo-treated patients, but the differences did not achieve statistical significance.

**Table 1: Therapeutic Effect and Sleep Questionnaire**

| | **ITT population** | | |
|---|---|---|---|
| | **Oxcarbazepine** | **Placebo** | ***P* value (vs placebo)** |
| **Sleep questionnaire** % days during double-blind phase, mean (SD) | n=68 | n=76 | |
| Delayed getting to sleep | 28 (31) | 38 (38) | 0.09 |
| Awaken from sleep | 31 (29) | 49 (38) | 0.02 |
| Felt rested after sleep | 55 (34) | 46 (37) | 0.17 |

| | | | |
|---|---|---|---|
| Abbreviations: ITT, intent-to-treat; SD, standard deviation | | | |

## Claims

1. The use of oxcarbazepine for the manufacture of a pharmaceutical composition for the improvement of sleep in human patients suffering from chronic pain.

2. The use according to claim 1 wherein the pain is diabetic neuropathic pain.

3. The use according to claim 1 or 2 wherein oxcarbazepine is administered at a total dose between about 450 mg/day and about 2100 mg/day.

4. The use according to any one of claims 1 to 3 wherein oxcarbazepine is administered on a twice daily schedule.

5. The use according to any one of claims 1 to 3 wherein the patient is suffering from neuropathic pain.

6. The use according to any one of claims 1 to 3 wherein the patient recruits from the Caucasian population.

7. The use according to any one of claims 1 to 3 wherein the sleep is Improved by decreasing the frequency of being awakened from sleep due to pain.

8. The use according to any one of claims 1 to 3 wherein the sleep is improved by decreasing the delay of getting to sleep due to pain.

9. The use according to any one of claims 1 to 3 wherein the sleep is improved by feeling rested after sleep.

10. A pharmaceutical composition comprising as sole active ingredient oxcarbazepine for use in the improvement of sleep in human patients suffering from chronic pain.

## Patentansprüche

1. Verwendung von Oxcarbazepin für die Herstellung einer pharmazeutischen Zubereitung zur Verbesserung des Schlafs in menschlichen Patienten, die an chronischem Schmerz leiden.

2. Verwendung nach Anspruch 1, worin der Schmerz Diabetis-Neuropathie-Schmerz ist.

3. Verwendung nach Anspruch 1 oder 2, worin Oxcarbazepin in einer Gesamtdosis zwischen etwa 450 mg/Tag und etwa 2.100 mg/Tag verabreicht wird.

4. Verwendung nach einem der Ansprüche 1 bis 3, worin Oxcarbazepin in einem Verabreichungsplan zweimal täglich verabreicht wird.

5. Verwendung nach einem der Ansprüche 1 bis 3, worin der Patient an neuropathischem Schmerz leidet.

6. Verwendung nach irgendeinem der Ansprüche 1 bis 3, worin der Patient aus der kaukasischen Population stammt.

7. Verwendung nach irgendeinem der Ansprüche 1 bis 3, worin der Schlaf verbessert wird durch Senken der Häufigkeit des Aus-dem-Schlaf-aufgeweckt-Werdens aufgrund von Schmerzen.

8. Verwendung nach irgendeinem der Ansprüche 1 bis 3, worin der Schlaf verbessert wird durch Senken der Schmerz-bedingten Verzögerung, einschlafen zu können.

9. Verwendung nach irgendeinem der Ansprüche 1 bis 3, worin der Schlaf verbessert wird durch ein Fühlen des Ausgeruht-Seins nach dem Schlafen.

10. Pharmazeutische Zubereitung, umfassend als einzigen aktiven Wirkstoff Oxcarbazepin zur Verwendung bei der Verbesserung des Schlafes in menschlichen Patienten, die an chronischem Schmerz leiden.

## Revendications

1. Utilisation de l'oxcarbazépine pour la fabrication d'une composition pharmaceutique pour l'amélioration du sommeil dans des patients humains souffrant d'une douleur chronique.

2. Utilisation selon la revendication 1, dans laquelle la douleur est une douleur neuropathique diabétique.

3. Utilisation selon l'une des revendications 1 ou 2, dans laquelle l'oxcarbazépine est administrée à une dose totale entre environ 450 mg/jour et environ 2 100 mg/jour.

4. Utilisation selon l'une quelconque des revendications 1 à 3, dans laquelle l'oxcarbazépine est administrée sur un programme de deux fois par jour.

5. Utilisation selon l'une quelconque des revendications 1 à 3, dans laquelle le patient souffre de douleur neuropathique.

6. Utilisation selon l'une quelconque des revendications 1 à 3, dans laquelle le patient vient de la population caucasienne.

7. Utilisation selon l'une quelconque des revendications 1 à 3, dans laquelle le sommeil est amélioré par diminution de la fréquence de réveil pendant le sommeil en raison de la douleur.

8. Utilisation selon l'une quelconque des revendications 1 à 3, dans laquelle le sommeil est amélioré par diminution du retard à s'endormir en raison de la douleur.

9. Utilisation selon l'une quelconque des revendications 1 à 3, dans laquelle le sommeil est amélioré par sensation de repos après le sommeil.

10. Composition pharmaceutique comprenant comme seul ingrédient actif de l'oxcarbazépine en vue d'une utilisation dans l'amélioration du sommeil dans des patients humains souffrant de douleur chronique.
